# EUROPEAN PATENT APPLICATION

(11) **EP 2 945 368 A1**
(43) Date of publication of application: **18.11.2015**
(21) Application number: 15167811.7
(22) Date of filing: 15.05.2015
(51) Int. Cl.: H04N 5/235, H04N 5/243, H04N 5/232

(54) **APPARATUS AND METHOD FOR OBTAINING VITAL SIGN OF SUBJECT**

(30) Priority: 16.05.2014 US 201461994237 P; 11.05.2015 US 201514708301
(71) Applicant: MediaTek, Inc, Hsin-Chu 300 (TW)
(72) Inventor: CHEN, Tung-Chien, 105 Taipei City (TW); CHEN, Yu-Ting, 110 Taipei City (TW); HUANG, Ying-Yuan, 302 Hsinchu County (TW)
(74) Representative: Hoefer & Partner Patentanwälte mbB

(57) **Abstract**

An apparatus (100) for obtaining a vital sign of a subject incudes an image sensor (120) for capturing a video of the subject to generate a plurality of first video frames (FRA_1-FRA_N); and a vital sign processor (110) for processing the plurality of first video frames (FRA_1- FRA_N) to generate a vital sign signal. The vital sign processor (110) adjusts a first parameter set for configuring the image sensor (120) to make the plurality of first video frames (FRA_1- FRA N) suitable for obtaining the vital sign of the subject.

## Description

The present invention relates generally to image processing, and more particularly, to an apparatus and method of obtaining a vital sign of a subject based on unprocessed/uncompressed image data.

State-of-the-art electronic systems, such as smartphones, tablets, touch-controlled devices, laptops, or personal computers, have a variety of built-in sensors, and these electronic systems can extend their functionalities by running a variety of applications using information provided by the built-in sensors. For example, with an image sensor capturing an image of human, the electronic systems could perform vital sign calculation to obtain a vital sign of the human. By running an application to analyze color changes in skin-tone regions of the image of the human, it is possible to calculate a heart rate of the human.

In most electronic systems, an application is only allowed to obtain processed/compressed image data, such as, YUV 420 image or H.264 video from an image signal processor or a video encoder coupled to the image sensor. Hence, the vital sign related applications in the electronic system only could obtain the compressed/processed image data.

In view of this, the accuracy of vital sign calculation is limited by color information reserved in processed/compressed image. However, the processed/compressed image is basically optimized for human's visual perception. For example, the processed/compressed image could be brighter, more contrast, and more saturated than raw image outputted by the image sensor 10. In addition, the compressed image data could even discard some details that are regarded as not perceptible to human's eye. Hence, there may be some color information lost/discarded during processing/compression. However, this information may be critical and useful in vital sign calculation. Therefore, the compression/processing of the image data may disadvantageously affect the accuracy of the vital signal calculation and is unfavorable to the vital signal calculation.

Accordingly, the invention aims at providing a method that can improve the accuracy of the vital sign calculation. The method acquires the unprocessed/uncompressed image data from the image sensor. Hence, colors changes in skin-tone regions can be reserved as possibly. In addition, the invention uses different image signal processing parameters to make image data respectively suitable for vital sign calculation and visualization to give consideration both to the accuracy of vital sign calculation and good user's visual perception. In addition, to reduce the bandwidth needed for transmission of uncompressed image data, the invention uses a frame sequence having reduced-size and reduced-frame rate extracted from raw output of the image sensor.

As will be seen more clearly from the detailed description following below, an apparatus for obtaining a vital sign of a subject is described. The apparatus includes: an image sensor and a vital sign processor. The image sensor is employed for capturing a video of the subject to generate a plurality of first video frames. The vital sign processor is employed for processing the plurality of first video frames to generate a vital sign signal. Additionally, the vital sign processor adjusts a first parameter set for configuring the image sensor to make the plurality of first video frames suitable for obtaining the vital sign of the subject.

As will be seen more clearly from the detailed description following below, a method for obtaining a vital sign of a subject is described. The method includes acquiring a plurality of first video frames of a video of the subject from an image sensor; processing the plurality of first video frames to generate a vital sign signal; and adjusting a first parameter set for configuring the image sensor to make the plurality of first video frames suitable for obtaining the vital sign of the subject.

In the following, the invention is further illustrated by way of example, taking reference to the accompanying drawings. Thereof
FIG. 1 is a diagram illustrates a vital sign calculation part of a conventional electronic system.
FIG. 2 is a diagram illustrates an apparatus of obtaining a vital sign of a subject according to one embodiment of the present invention.
FIG. 3 is a diagram illustrates an underexposed image captured in a backlight scene.
FIG. 4 is a flowchart regarding adjusting the first parameter set.
FIG. 5 is a diagram illustrating how to adjust the first parameter set in further details.
FIG. 6 is a flowchart regarding interpolating missing data according to one embodiment of the present invention.
FIG. 7 is a diagram illustrating a layout of combining vital sign information and visualization of the subject.
FIG. 8 is a flowchart of a method of obtaining a vital sign of a subject according to one embodiment of the present invention.

Certain terms are used throughout the following descriptions and claims to refer to particular system components. As one skilled in the art will appreciate, manufacturers may refer to a component by different names. This document does not intend to distinguish between components that differ in name but not differ in functionality. In the following discussion and in the claims, the terms "include", "including", "comprise", and "comprising" are used in an open-ended fashion, and thus should be interpreted to mean "including, but not limited to ..." The terms "couple" and "coupled" are intended to mean either an indirect or a direct electrical connection. Thus, if a first device couples to a second device, that connection may be through a direct electrical connection, or through an indirect electrical connection via other devices and connections.

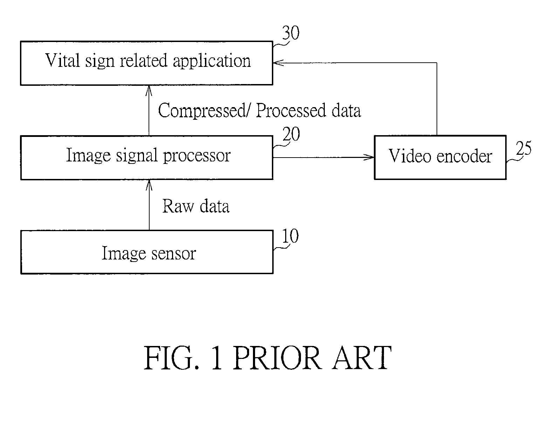

FIG. 1 illustrates how a vital sign calculation works under a conventional electronic system. As shown by FIG. 1, raw data outputted by the image sensor 10 will be processed/compressed by the image signal processor 20 or the video encoder 25, and transformed into image data of YUV format (e.g., YUV420, YUV444 and YUV422) or H.264 format. Hence, the vital sign related application 30 only could obtain the compressed/processed image data. The accuracy of the vital sign calculation is therefore limited.

### Vital Sign Processor

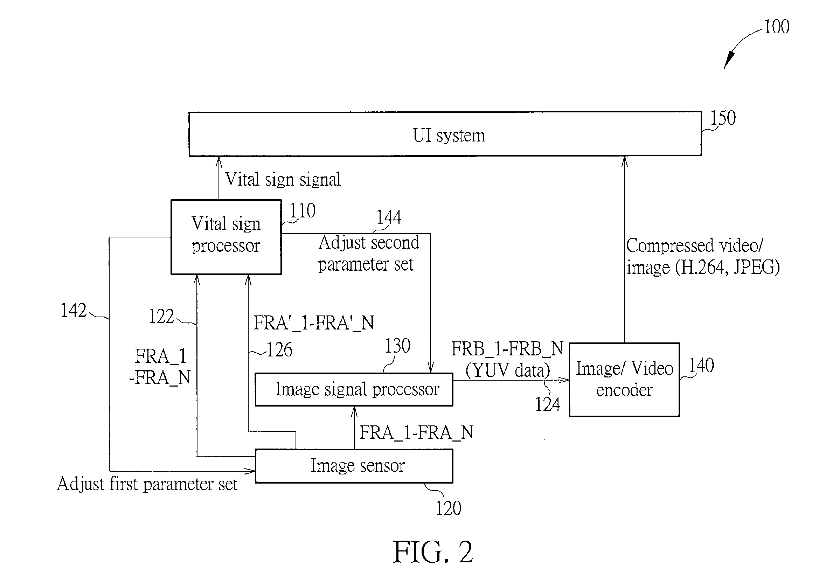

Please refer to FIG. 2, which illustrates apparatus 100 including a vital sign processor 110 according to one embodiment of the present invention. The apparatus 100 could be a part or a subsystem of an electronic system. The electronic system could be a smartphone, a tablet, a touch-controlled device, a laptop, or a personal computer. An image sensor 120 of the apparatus 100 is employed for capturing a video of a subject (which could be one or multiple humans' or animals' bodies). The vital sign processor 110 acquires first video frames FRA_1- FRA_N of the video and processes them to generate a vital sign signal to indicate a vital sign of the subject. According to various embodiments of the present invention, the vital sign processor 110 could be implemented with a general-purpose processor or a dedicated hardware, and the vital sign could be blood pressure, heart rate, blood oxygen saturation, and/or heart rate variability of the subject. In addition, the image sensor 120 could be charge coupled device (CCD) image sensor or a complementary metal oxide semiconductor (CMOS) image sensor. When the image sensor 120 is a CMOS image sensor, the vital sign processor 110 and the image sensor 120 could be integrated on a same chip, e. g. system on a chip (SoC) ; otherwise, they could be two separated chips.

The first video frames FRA_1- FRA_N is directly outputted by the image sensor 120 through an interface 122 without being processed or compressed by an image signal processor (ISP) 130. The first video frames FRA_1- FRA_N could be in form of raw data. The format of first video frames FRA_1- FRA_N could be RBGB, RGBW, CYGM, WWWW, RGBIR, YUV formats or other formats of raw data. In other embodiments, the vital sign processor 110 could also use the image data from the ISP 130 in format of YUV422, YUV444 and so on, to generate the vital sign signal.

As each pixel in each of the first video frames FRA_1-FRA_N includes at least 10-bit (even could be 12-bit or 14-bit) information for each color component due to in form of raw data, when compared to the conventional YUV420 image data, the first video frames FRA_1- FRA_N could reserve more color information of the subject and has a higher dynamic range. Therefore, the first video frames FRA_1- FRA_N reserve colors changes of the subject to the greatest extent. This is useful for vital sign calculation.

In one embodiment, in order to save a bandwidth of an interface between the vital sign processor 110 and the image sensor 120, the vital sign processor 110 could use fewer frames from the first video frames FRA_1- FRA_N or uses a portion of a whole of each first video frame for vital sign calculation. That is, the vital sign processor 110 performs the vital signal calculation based on frames of reduced-size and/or reduced-frame rate. In this embodiment, the image sensor 120 could send a plurality of video frames FRA'_1- FRA'_N of reduced-size and reduced-frame rate (compared to the first video frames FRA_1- FRA_N) to the vital sign processor 110 via an interface 126. For example, if the first video frames FRA_1- FRA_N have a frame size of 1280x720, the vital sign processor 110 could acquire a reduced-sized video frames FRA'_1- FRA'_N, each having a frame size of 640x360 from the interface 126 and processes 640x360 pixel data which is actually out of pixel data of a full frame of the first video frames FRA_1- FRA_N to calculate the vital sign. Furthermore, if the first video frames FRA_1- FRA_N have a frame rate at 30fps, the vital sign processor 110 could acquire the video frames FRA'_1- FRA'_N at 10 fps transmitted on the interface 126, which is actually comprised of one out of every three of the first video frames FRA_1- FRA_N, and processes them to calculate the vital sign. Compared to the interface 122, the bandwidth needed by the interface 126 is much smaller since it transmits the reduced-size and reduced-frame rate video frames FRA'_1- FRA'_N. Please note that, the aforementioned numbers of reduced-frame rate and reduced-size is just for illustration rather than limitations.

### Configuring Image Sensor

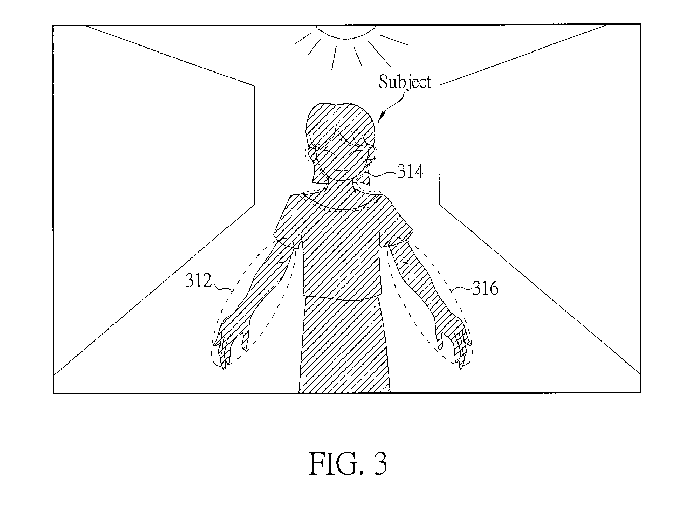

The vital sign processor 110 could include two parts with different functionalities, one of which is for pre-processing the first video frames FRA_1- FRA_N (or video frames FRA'_1-FRA'_N) to have the image data optimized for vital sign calculation while the other of which is for performing vital sign calculation. During the pre-processing, the vital sign processor 110 configures the image sensor 120 to make the first video frames FRA_1- FRA_N suitable for obtaining the vital sign. For example, in a backlighted scene as shown by FIG. 3, the subject is underexposed. Therefore, the vital sign processor 110 may fail to acquire all the details on the subject. Hence, the vital sign processor 110 needs to control the image sensor 120 to expose the subject properly, in the backlighted scene or other specific scene that has complicated light sources, in consideration of vital sign calculation.

The vital sign processor 110 can adjust a first parameter set to configure the image sensor 120 through a control path 142. In various embodiments of the preset invention, the first parameter set could comprise one of an exposure control parameter and a gain control parameter or both. The exposure control parameter could determine an exposure value (EV) for the image sensor 120 to generate the first video frames FRA_1-FRA_N, which involves shutter speed and lens aperture. The gain control parameter could determine the signal gain of the image sensor 120, which would be the level of ISO sensitivity at which the image sensor 120 is configured to generate first video frames FRA_1- FRA_N.

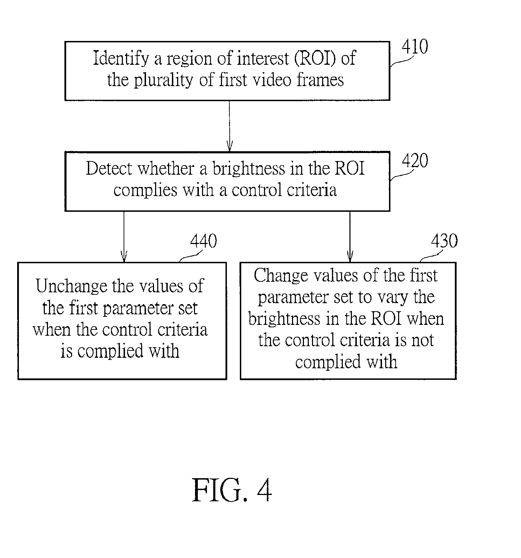

FIG. 4 illustrates a flow regarding adjusting the first parameter set to configure the image sensor 120. At first, a region of interest (ROI) of the plurality of first video frames FRA_1- FRA_N is identified. Taking the video frame shown by FIG. 3 as an example, regions 312, 314 and 316 which is regarded as human's skin will be identified as the ROI of the video frames FRA_1- FRA_N. After the ROI has been identified, the vital sign processor 110 detects whether brightness in the ROI complies with a control criteria. If the brightness in the ROI does not comply with the control criteria, the vital sign processor 110 changes the values of the first parameter set to vary the brightness. If the brightness in the ROI is complied with the control criteria, the vital sign processor 110 does not change the values of the first parameter set.

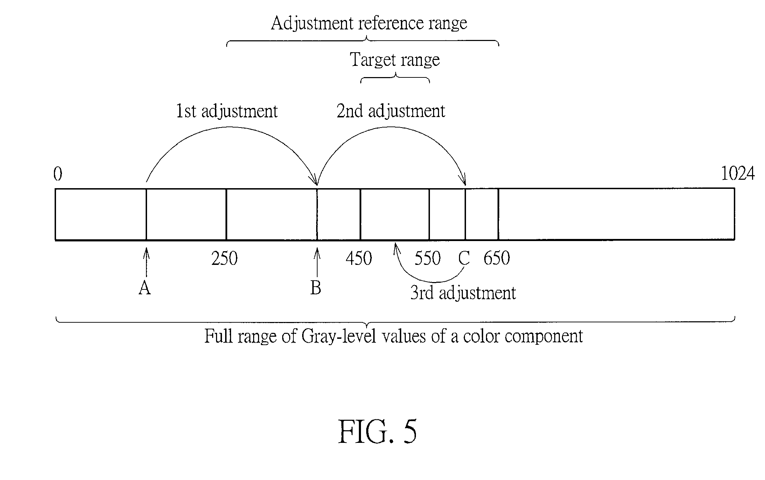

FIG. 5 illustrates how the adjustment works in further details according to one embodiment of the present invention. After the ROI of a video frame is identified, the vital sign processor 110 further calculates an average of gray-level values of a specific color component corresponding to pixels within the identified ROI (hereinafter as "the average within the ROI"). In this embodiment, the vital sign processor 110 detects whether the average within the identified ROI falls within an adjustment reference range (250-650) of a full range of gray-level value of the color component (which would be 0-1024 if the image sensor 120 outputs 10-bit color information) . If no, the vital sign processor 110 adjusts the values of the first parameter set. For example, the vital sign processor 110 could adjust one of the exposure control parameter and the gain control parameter to configure the image sensor 120. After adjustment, the image sensor 120 generates a following first video frame according to the adjusted first parameter set. The goal of the adjustment on the first parameter set is to control the average within the ROI of the following first video frame fall within a target range (450-550). Hence, the vital sign processor 110 will repeatedly adjust values of the first parameter set over following first video frames until it is detected the average within ROI falling within the adjustment reference range. In the above descriptions, the adjustment reference range is longer than the target range in interval such that the adjustment does not need to be performed usually. However, this is only for the purpose of illustration, rather than limitations. In other embodiments, the adjustment reference range could be shorter than or equal to the target range in interval. In addition, it is also possible to adjust the first parameter set if it is detected that the scene changes or the vital sign cannot be obtained for a long time.

In addition, FIG. 5 also illustrates a possible algorithm to control the average within the ROI to comply with the control criteria according to one embodiment of the present invention. It is assumed that the average is initially A, which does not fall within the adjustment reference range. Therefore, the vital sign processor 110 starts to adjust the values of the first parameter set. As the value A is lower than upper bound 550 and lower bound 450 of the target range, the vital sign processor 110 adjusts the first parameter set to have the image sensor 120 able to get a brighter first video frame (e.g. configuring the image sensor 120 to have higher EV or higher ISO sensitivity) . Accordingly, the image sensor 120 generates the following first video frame based on the adjusted first parameter set. Then, the vital sign processor 110 again detects the average within the ROI of the following first video frame to be B, which is still lower than upper bound 550 and lower bound 450 of the target range, the vital sign processor 110 again adjusts the first parameter set to have the image sensor 120 able to get a much brighter first video frame. Finally, it is detected the average within the ROI of the following first video frame is C, which becomes higher than upper bound 550 and lower bound 450 of the target range, and the vital sign processor 110 adjusts the first parameter set to have the image sensor 120 able to get a darker first video frame (e.g. configuring the image sensor 120 to have lower EV or lower ISO sensitivity) than previous one. Accordingly, the vital sign processor 110 stops adjusting the first parameter set. In one embodiment, each time the vital sign processor 110 adjusts the first parameter set, the vital sign processor 110 could change the value of only one parameter, while remain other parameters unchanged. For example, the vital sign processor 110 could change the value of the exposure control parameter while remain the value of gain control parameter unchanged, or vice versa. In addition, in one embodiment, the vital sign processor 110 may not immediately adjust the first parameter set once it is detected that the average within ROI does not fall within the adjustment reference range. The vital sign processor 110 could wait for a couple of first video frames and then to detect the average within the ROI of the first video frame.

In one embodiment, the vital sign processor 110 could calculate the average of gray-level values of the green color components corresponding to the pixels within the ROI to determine whether to adjust the first parameter set. However, this is not intended to be limitations of the present invention. According to various embodiments of the present invention, the vital sign processor 110 could calculate the average based on values corresponding to other color components of the pixels within the ROI.

### Missing data compensation

In some conditions, the image sensor 120 may not provide the first video frames FRA_1-FRA_N at a steady and fixed frame rate due to possible delay. Hence, some frames may drop. This could disadvantageously affect accuracy of the vital sign calculation.

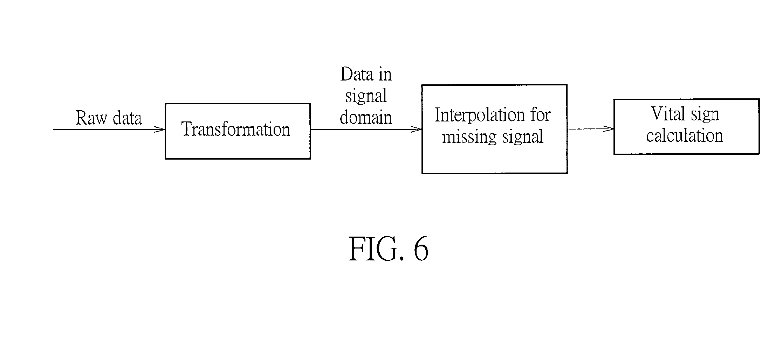

To address this problem, the vital sign processor 110 could perform a frame rate adjustment to interpolate the missing data corresponding to the dropped frames. Specifically, the vital sign processor 120 will transform the data in video frames into a signal domain. For example, pixel data of the ROI of the video frame will be transformed to an average value of luminance of pixels within the ROI. Due to frame dropping, there are some average values missing. The vital sign processor 110 performs data interpolation to compensate the missing data in a data sequence. Then, vital sign processor 110 could use the compensated data sequence in signal domain to perform the vital sign calculation. In one embodiment, the image sensor 120 could generate a timestamp corresponding to a video frame each time the video frame is generated. The vital sign processor 110 performs frame rate adjustment (i.e., compensation), according to the plurality of time stamps, with respect to the plurality of first video frames FRA_1-FRA_N to generate the vital sign signal. In other embodiment, the vital sign processor 110 generates a plurality of time stamps according to a system clock, wherein each of the plurality of time stamps is associated with each of the plurality of first video frames. Then, the vital sign processor 110 performs frame rate adjustment, according to the plurality of time stamps, with respect to the plurality of first video frames FRA_1-FRA_N to generate the vital sign signal. A simplified flow of compensating missing data is illustrated in FIG. 6.

### Image signal Processor

In the apparatus 100, the ISP 130 is employed for processing the first video frames FRA_1- FRA_N according to a second parameter set, to provide a plurality of second video frames FRB_1- FRB_N for visualization of the subject. The second video frames FRB_1- FRB_N could have a YUV format. The second video frames FRB_1- FRB_N will then be sent via interface 124 to an UI system 150 for displaying on a display device of the electronic system. Alternatively, the second video frames FRB_1- FRB_N is also accessible by other hardware or software on the system for other purposes.

As the second video frames FRB_1- FRB_N is for visualization of the subject, the second video frames FRB_1-FRB_N needs to be favorable to human's eye. For example, the second video frames FRB_1- FRB_N could be brighter, sharper, more contrast, and/or more saturated. In view of this, the second parameter set could comprise (but not limited to) a contrast parameter, a saturation parameter, a sharpness parameter, a hue parameter, a gamma parameter, a backlight parameter, or a power line frequency parameter. The image signal processor 130 adjusts the second parameter set through a control path 144 to get a good user's visual perception.

In addition, the second video frames FRB_1- FRB_N could be further outputted to a video/image encoder 140 to be compressed based on a specific video/image compression algorithm to generate a compressed video/image, for example, H.264 video or JPEG image.

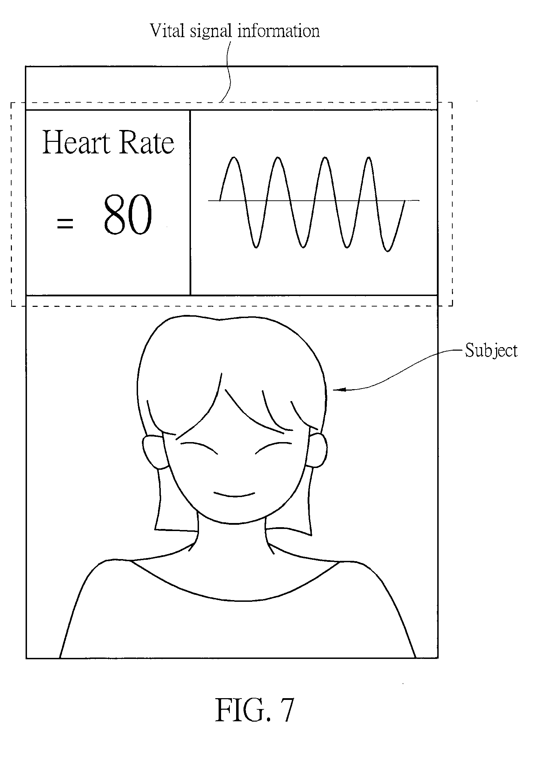

Both of the vital sign signal generated by the vital sign processor 110 and the second video frames FRB_1-FRB_N (or corresponding compressed H.264 video/JPEG image) that are optimized for visualization are sent to the UI system 150. The UI system 150 could overlay the vital sign information indicated by the vital sign signal over the second video frames FRB_1-FRB_N (or corresponding compressed H.264 video/JPEG image) of the subject to present the visualization of the subject and the vital sign information in a single frame (as shown by FIG. 7). As the UI system 150 combines the second video frames FRB_1-FRB_N (or corresponding compressed H.264 video/JPEG image) is optimized for visualization (because it is processed by the second parameter set) and the vital sign information is obtained based on the image data that reserves most color changes. The present invention therefore realizes a good vital sign extraction and display mechanism.

### Flow of obtaining a vital sign

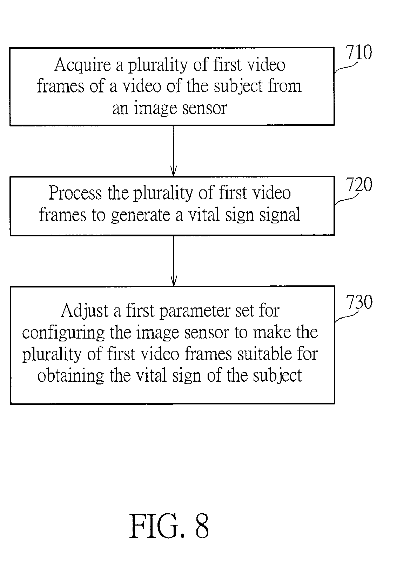

FIG. 8 illustrates a flow of a method of obtaining a vital sign of a subject according to one embodiment of the present invention. The flow include following steps:
Step 710: Acquire a plurality of first video frames of a video of the subject from an image sensor;
Step 720: Process the plurality of first video frames to generate a vital sign signal; and
Step 730: Adjust a first parameter set for configuring the image sensor to make the plurality of first video frames suitable for obtaining the vital sign of the subject.

Principles, detailed implementations, and modification regarding Steps 710-730 have been illustrated in explanation of the apparatus 100. Therefore, detailed descriptions regarding the Steps 710-730 are omitted here for the sake of brevity.

To sum up, the present invention provides a way of achieving high accuracy and good user's visual perception based on acquiring raw output of the image sensor and different output channels of the image sensor for different purposes. Also, using data interpolation technique to address the problem caused by frame dropping can improve the accuracy of the vital sign calculation, and using frames of reduced-sized and reducing-frame rate can reduce the needed bandwidth of the interface for interfacing the vital sign processor with the image sensor, which leads to a vital sign obtaining device of high performance and low cost.

Reference in the specification to "one embodiment" or "an embodiment" means that a particular feature, structure, or characteristic described in connection with the embodiment is included in at least an implementation. The appearances of the phrase "in one embodiment" in various places in the specification are not necessarily all referring to the same embodiment. Thus, although embodiments have been described in language specific to structural features and/or methodological acts, it is to be understood that claimed subject matter may not be limited to the specific features or acts described. Rather, the specific features and acts are disclosed as sample forms of implementing the claimed subject matter.

Examples of the hardware in the present invention may include analog circuit(s), digital circuit(s) and/or mixed circuit(s). For example, the hardware may include ASIC(s), field programmable gate array(s) (FPGA(s)), programmable logic device(s), coupled hardware components or combination thereof. In another example, the hardware may include general-purpose processor(s), microprocessor(s), controller(s), digital signal processor(s) (DSP(s)) or combination thereof. The electronic system mentioned above could be included a system on chip (SOC), system in package (SiP).

## Claims

1. An apparatus (100) for obtaining a vital sign of a subject, **characterized by**:
an image sensor (120), for capturing a video of the subject to generate a plurality of first video frames (FRA_1-FRA_N); and
a vital sign processor (110), for processing the plurality of first video frames (FRA_1- FRA_N) to generate a vital sign signal,
wherein the vital sign processor (110) adjusts a first parameter set for configuring the image sensor (120) to make the plurality of first video frames (FRA_1-FRA_N) suitable for obtaining the vital sign of the subject.

2. The apparatus (100) of claim 1, **characterized in that** the vital sign processor (110) processes one out of every N of the plurality of first video frames (FRA_1- FRA_N) to generate the vital sign signal, wherein N is a positive integer greater than 1.

3. The apparatus (100) of claim 1, **characterized in that** the first parameter set comprises one of an exposure control parameter and a gain control parameter.

4. The apparatus (100) of claim 1, **characterized in that** the vital sign processor (110) adjusts the first parameter set for configuring the image sensor (120) by:
identifying a region of interest, hereinafter ROI, of the plurality of first video frames (FRA_1- FRA_N) ; detecting whether a brightness in the ROI complies with a control criteria; and
changing values of the first parameter set to vary the brightness in the ROI when the control criteria is not complied with; or
unchanging the values of the first parameter set when the control criteria is complied with.

5. The apparatus (100) of claim 4, **characterized in that** the control criteria is whether an average of gray-level values of a color component corresponding to the plurality of first video frames (FRA_1- FRA_N) is within specific range.

6. The apparatus (100) of claim 1, **characterized by**:
an image signal processor (130), hereinafter ISP, for processing the plurality of first video frames (FRA_1- FRA_N) according to a second parameter set, to provide a plurality of second video frames (FRB_1-FRB_N) for visualization of the subject,
wherein the second parameter set comprises a contrast parameter, a saturation parameter, a sharpness parameter, a hue parameter, a gamma parameter, a backlight parameter, or a power line frequency parameter.

7. The apparatus (100) of claim 6, **characterized in that** wherein a frame format of the plurality of first video frames (FRA_1- FRA_N) is different from a frame format of the plurality of second video frames (FRB_1- FRB_N).

8. The apparatus (100) of claim 1, **characterized in that** the image sensor (120) further generates a plurality of time stamps corresponding to the plurality of first video frames (FRA_1- FRA_N) and the vital sign processor (110) performs frame rate adjustment, according to the plurality of time stamps, with respect to the plurality of first video frames (FRA_1- FRA_N) to generate the vital sign signal.

9. The apparatus (100) of claim 1, **characterized in that** the vital sign processor (110) : generates a plurality of time stamps, wherein each of the plurality of time stamps is associated with each of the plurality of first video frames (FRA_1- FRA_N); and
performs frame rate adjustment, according to the plurality of time stamps, with respect to the plurality of first video frames (FRA_1- FRA_N) to generate the vital sign signal.

10. A method for obtaining a vital sign of a subject, **characterized by**:
acquiring a plurality of first video frames (FRA_1- FRA_N) of a video of the subject from an image sensor (120) ;
processing the plurality of first video frames (FRA_1-FRA_N) to generate a vital sign signal; and
adjusting a first parameter set for configuring the image sensor (120) to make the plurality of first video frames (FRA_1- FRA_N) suitable for obtaining the vital sign of the subject.

11. The method of claim 10, **characterized in that** the step of generating the vital sign signal comprises:
processing one out of every N of the plurality of first video frames (FRA_1- FRA_N) to generate the vital sign signal, wherein N is a positive integer greater than 1.

12. The method of claim 10, **characterized in that** first parameter set comprises one of an exposure control parameter and a gain control parameter.

13. The method of claim 10, **characterized in that** the step of adjusting the first parameter set for configuring the image comprises:
identifying a region of interest, hereinafter ROI, of the plurality of first video frames (FRA_1- FRA_N) ;
detecting whether a brightness in the ROI complies with a control criteria; and
changing values of the first parameter set to vary the brightness in the ROI when the control criteria is not complied with; or
unchanging the values of the first parameter set when the control criteria is complied with.

14. The method of claim 13, **characterized in that** the control criteria is whether an average of gray-level values of a color component corresponding to the plurality of first video frames (FRA_1- FRA_N) is within a specific range.

15. The method of claim 10, **characterized by**:
processing the plurality of first video frames (FRA_1-FRA_N) according to a second parameter set, to provide a plurality of second video frames (FRB_1- FRB_N) for visualization of the subject,
wherein the second parameter set comprises a contrast parameter, a saturation parameter, a sharpness parameter, a hue parameter, a gamma parameter, a backlight parameter, or a power line frequency parameter.
